# EUROPÄISCHE PATENTSCHRIFT

(11) **EP 2 900 209 B1**
(45) Veröffentlichungstag und Bekanntmachung des Hinweises auf die Patenterteilung: **21.10.2020**
(21) Anmeldenummer: 13715155.1
(22) Anmeldetag: 22.03.2013
(51) Int. Cl.: A61K 9/00, A61K 31/198, A61K 31/375, A61K 31/4415, A61K 31/519, A61K 31/525, A61K 31/714, A61K 33/30, A61K 45/06, A61P 31/22

(54) **LYSINHALTIGE ZUSAMMENSETZUNG ZUR NUTRITIVEN ERGÄNZUNG ODER BEHANDLUNG BEI HERPES**
COMPOSITION CONTAINING LYSINE AS A NUTRITIONAL SUPPLEMENT OR TREATMENT IN THE CASE OF HERPES
COMPOSITION À TENEUR EN LYSINE EN TANT QUE COMPLÉMENT ALIMENTAIRE OU POUR LE TRAITEMENT DE L'HERPÈS

(30) Priorität: 28.09.2012 DE 202012103733 U
(43) Veröffentlichungstag der Anmeldung: 05.08.2015
(73) Patentinhaber: Rittinghausen, Reiner, 82266 Inning-Bachern (DE)
(72) Erfinder: Rittinghausen, Reiner, 82266 Inning-Bachern (DE)
(74) Vertreter: Michalski Hüttermann & Partner Patentanwälte mbB
(86) Internationale Anmeldenummer: PCT/EP2013/056145
(87) Internationale Veröffentlichungsnummer: WO 2014/048584

(56) Entgegenhaltungen:
- WO-A1-03/035027
- DE-A1- 10 221 403
- US-B2- 6 455 061
- FLODIN N W: "The metabolic roles, pharmacology, and toxicology of lysine", JOURNAL OF THE AMERICAN COLLEGE OF NUTRITION, AMERICAN COLLEGE OF NUTRION, WILMINGTON, NC, US, Bd. 16, Nr. 1, 1. Februar 1997 (1997-02-01), Seiten 7-21, XP009103689, ISSN: 0731-5724
- FRAUKE BARKHOFF: "Hilfe bei Herpesinfektionen und Gürtelrose", NEUE WEGE ZUR GESUNDHEIT, CONSTANTIA VERLAG, Bd. 15, Nr. 7, Juli 2001 (2001-07), Seiten 1-2, XP002704070, Leer, Germany

## Beschreibung

Herpes ist eine Infektionskrankheit, die durch Viren ausgelöst wird. Es gibt viele verschiedene Herpes-Viren. Zu den humanpathogenen Herpesviren gehören die Herpes simplex-Viren Herpes-Virus Typ 1 (HSV-1) und Herpes-Virus Typ 2 (HSV-2), das insbesondere Herpes der Geschlechtsorgane (Herpes genitalis) verursacht. Weiter gehören zu den humanpathogenen Herpesviren das Varicella Zoster-Virus, das Windpocken oder Gürtelrose (Herpes zoster) verursacht, das Epstein-Barr-Virus, das Pfeiffersches Drüsenfieber verursacht, das Cytomegalie-Virus, das die Speicheldrüsenkrankheit verursacht, sowie das humane Herpes-Virus Typ 6 (HHV-6), das Drei-Tage-Fieber verursacht.

Insbesondere das Herpes-simplex-Virus Typ 1 (HSV-1) ist weit verbreitet. Es wird geschätzt, dass etwa 90 % der mitteleuropäischen Bevölkerung mit dem Herpes-Virus Typ 1 infiziert sind. Die Erstinfektion erfolgt zumeist schonwährend der ersten Lebensjahre. Diese verläuft häufig latent, manche Kinder erkranken jedoch an der so genannten Mundfäule (Gingivostomatitis herpetica). Da die Viren latent im Körper verblieben, kommt es bei ca. 15 % bis 30 % der Infizierten in den späteren Lebensjahren immer wieder zum Ausbruch des Lippenherpes (Herpes labialis). Auslösende Faktoren können Erkältung und Fieber, körperliche Erschöpfung, Stress, hormonelle Schwankungen, Sonneneinstrahlung oder Hitzeeinwirkung sowie Ekelgefühle sein.

Es ist derzeit kein Wirkstoff bekannt, der in der Lage wäre, Herpes-Viren im Körper abzutöten bzw. aus diesem zu entfernen. Übliche Behandlungsmethoden von Lippenherpes beinhalten das Auftragen von Lippencremes, die chemische Wirkstoffe enthalten, die die RD 41562 / SAM:AL

Vermehrung des Virus hemmen sollen (Virustatika), beispielsweise Aciclovir. Auch Cremes oder Gele mit Zinksulfat, das ebenfalls antiviral wirkt, sind bekannt. Jedoch kann die topische Anwendung von Zinklösungen schmerzhafte oder reizende Nebenwirkungen hervorrufen.

In der Literatur finden sich weiterhin Hinweise zur Verabreichung von Vitamin C, Zink oder der Aminosäure L-Lysin als vorbeugende Maßnahme bzw. beim Auftreten erster Symptome. Hierbei wirkt Vitamin C abwehrsteigernd, während Zink immunstimulierend wirkt. Da das das Herpesvirus für seine Vermehrung die Aminosäure L-Arginin benötigt, sollen argininreiche Nahrungsmittel möglichst gemieden werden, während lysinreiche Lebensmittel wegen dessen Fähigkeit, die Aufnahme von Arginin zu hemmen, verstärkt verzehrt werden sollen. Obwohl eine lysinreiche und argininarme Ernährung eine vorbeugende Maßnahme gegen einen Ausbruch von Herpes darstellen kann, ist dies für eine Behandlung ungeeignet. Es besteht daher Bedarf an einem Mittel, das eine wirksame Behandlung von Lippen-Herpes zur Verfügung stellen kann.

US 6455061 B2 beschreibt Einheitsdosierungsformen für die Behandlung von Herpes simplex. WO 03/035027 A1 beschreibt kaubare Tabletten enthaltend Lysin. DE 102 21 403 A1 beschreibt diätetische und pharmazeutische Zusammensetzungen, ihre Herstellung und ihre Verwendung. N W Flodin beschreibt in J Am Coll Nutr. 1997, Vol. 16, Nr. 1, Seiten 7-21 "The metabolic roles, pharmacology, and toxicology of lysine". F. Barkhoff beschreibt in Neue Wege zur Gesundheit, Constantia-Verlag, Leer, Deutschland, Ausgabe Nr. 15, no. 7/2001, Seiten 1 - 2 "Hilfe bei Herpesinfektionen und Gürtelrose".

Es ist daher die Aufgabe der vorliegenden Erfindung, ein Mittel zur Verfügung zu stellen, das wenigstens einen der vorgenannten Nachteile des Standes der Technik überwindet. Insbesondere ist es die Aufgabe der vorliegenden Erfindung, ein Mittel zur Verfügung zu stellen, das zur Behandlung von Herpes-Infektionen verwendbar ist.

Die Aufgabe wird gelöst durch eine Zusammensetzung zur Verwendung in der prophylaktischen und/oder therapeutischen Behandlung von Lippenherpes, wobei die Zusammensetzung, bezogen auf eine Verabreichungseinheit der Zusammensetzung, als wirksame Bestandteile die nachstehenden Stoffe aufweist:
- L-Lysin im Bereich von ≥ 250 mg bis ≤ 3 g, und
- Zink im Bereich von ≥ 3 mg bis ≤ 30 mg, und
- Selen, Vitamin B₂, Vitamin B₆, Vitamin B₁₂, Vitamin C, Vitamin D₃, Folsäure und Bioflavonoide,
und in einer Darreichungsform ausgewählt aus der Gruppe umfassend Kautabletten, Kaudragees, Lutschtabletten, Pastille, Schmelztablette, Gel und/oder Kaugummi vorliegt, wobei die Darreichungsform gekaut, gelutscht oder im Mund zergehen gelassen wird, wobei die Wirkstoffe im Mundraum freigesetzt werden.

Überraschend hat sich gezeigt, dass die erfindungsgemäße Zusammensetzung die Dauer einer Herpes-Infektion verringern kann. Dies ist von großem Vorteil, da eine Herpes-Infektion von ihrem Ausbruch, beginnend mit Juckreiz, Kribbeln oder Schmerzgefühl an den Lippen, bis zum Abheilen der verkrusteten Bläschen in der Regel etwa zwei Wochen dauert. Darüber hinaus ist Lippenherpes schmerzhaft und für die Betroffenen auch aus ästhetischen Gründen eine große Belastung. Eine Verkürzung der Infektionsdauer um mehrere oder selbst einen Tag ist daher ein vordringliches Merkmal einer Behandlung. Es hat sich weiter überraschend gezeigt, dass die Gabe von L-Lysin und Zink gemäß dem erfindungsgemäßen Mengenbereich die Infektiosität von Herpesviren vermindern kann.

Weiterhin kann die erfindungsgemäße Zusammensetzung bei beginnendem Juckreiz oder Schmerzgefühl an den Lippen die Bläschenbildung unterdrücken. Zudem kann durch die erfindungsgemäße Zusammensetzung auch eine Prophylaxe von Herpes-Infektionen und/oder Entzündungen des Mund- und Rachenraums erzielt werden, bevor Symptome auftreten. So kann beispielsweise bei Anzeichen von Herpes-auslösenden Faktoren wie Erkältung oder Ekelgefühlen die Verabreichung der Zusammensetzung den Ausbruch bzw. die Häufigkeit von Herpes-Infektionen verhindern.

In vorteilhafter Weise können die erfindungsgemäßen Zusammensetzungen eine gute Wirkung in der nutritiven, prophylaktischen und/oder therapeutischen Behandlung von Herpes-Infektionen und/oder Entzündungen des Mund- und Rachenraums zeigen. Weiterhin konnte festgestellt werden, eine Verbesserung auch als Zusatztherapie zu anderen Herpes-Therapeutika festgestellt werden konnte.

Ein weiterer Gegenstand der Beschreibung betrifft entsprechend eine Zusammensetzung umfassend, bezogen auf eine Tagesdosis der Zusammensetzung, als wirksamen Bestandteil L-Lysin im Bereich von ≥ 750 mg bis ≤ 9 g zur Verwendung in der nutritiven Ergänzung oder prophylaktischen und/oder therapeutischen Behandlung von Herpes-Infektionen und/oder Entzündungen des Mund- und Rachenraums, insbesondere von Lippenherpes.

Ein noch weiterer Gegenstand der Beschreibung betrifft entsprechend eine Zusammensetzung die, bezogen auf eine Tagesdosis der Zusammensetzung, als wirksame Bestandteile die nachstehenden Stoffe, aufweist:
- L-Lysin im Bereich von ≥ 750 mg bis ≤ 9 g, und
- Zink im Bereich von ≥ 9 mg bis ≤ 90 mg,
und in einer Darreichungsform ausgewählt aus der Gruppe umfassend Kautabletten, Kaudragees, Lutschtabletten, Pastillen, Schmelztabletten, Gel, Dosierspray und/oder Kaugummi vorliegt, zur Verwendung in der nutritiven Ergänzung oder prophylaktischen und/oder therapeutischen Behandlung von Herpes-Infektionen und/oder Entzündungen des Mund- und Rachenraums, insbesondere von Lippenherpes.

Von besonderem Vorteil ist, dass eine schnelle Wirkung eintritt. Ein Ausbruch einer Herpes-Infektion macht sich durch Juckreiz oder Schmerzgefühl an den Lippen bemerkbar, bevor eine Bläschenbildung einsetzt. Überraschend wurde festgestellt, dass bei einer Einnahme der Zusammensetzung sobald Symptome auftraten, ein Ausbruch der Herpes-Infektion unterdrückt werden konnte. Besonders vorteilhaft ist hierbei, dass eine sofortige Wirkung eintrat und die Patienten bereits am folgenden Tag nahezu symptomfrei waren. Dies erlaubt insbesondere die Verabreichung der Zusammensetzung bei einer Akutbehandlung als eine einmalige und/oder mehrmalige, zeitlich begrenzte Verabreichung, die nicht vor dem Auftreten einer Herpes-Infektion begonnen oder längerfristig angewendet werden muss um eine Wirkung zu erzielen.

Ein weiterer Gegenstand der Beschreibung betrifft entsprechend eine Zusammensetzung umfassend, bezogen auf eine Tagesdosis der Zusammensetzung, als wirksamen Bestandteil L-Lysin im Bereich von ≥ 750 mg bis ≤ 9 g, zur Verwendung in der Akutbehandlung von Herpes-Infektionen und/oder Entzündungen des Mund- und Rachenraums, insbesondere in der Akutbehandlung von Lippenherpes. Von ganz besonderem Vorteil ist, dass bereits Lysin in der Akutbehandlung von Herpes-Infektionen insbesondere Lippenherpes eine gute Wirkung zeigen kann.

Ein noch weiterer Gegenstand der Beschreibung betrifft eine Zusammensetzung umfassend bezogen auf eine Tagesdosis der Zusammensetzung, als wirksame Bestandteile die nachstehenden Stoffe:
- L-Lysin im Bereich von ≥ 750 mg bis ≤ 9 g, und
- Zink im Bereich von ≥ 9 mg bis ≤ 90 mg,
und in einer Darreichungsform ausgewählt aus der Gruppe umfassend Kautabletten, Kaudragees, Lutschtabletten, Pastillen, Schmelztabletten, Gel, Dosierspray und/oder Kaugummi vorliegt, zur Verwendung in der Akutbehandlung von Herpes-Infektionen und/oder Entzündungen des Mund- und Rachenraums, insbesondere in der Akutbehandlung von Lippenherpes.

Unter einer "Akutbehandlung" im Sinne der vorliegenden Erfindung wird eine Verabreichung unter Erzielung einer vorteilhaften Wirkung zum Zeitpunkt bei oder nach Auftreten einer Erkrankung verstanden, ohne dass hierfür eine von Krankheitszuständen unabhängige, vorhergehende Langzeit-Verabreichung, d. h. über mehrere Tage, Wochen oder Monate, notwendig ist. Bei einer "Akutbehandlung" im Sinne dieser Erfindung tritt somit eine Linderung der Beschwerden ein, ohne dass hierfür eine von Krankheitszuständen unabhängige, prophylaktische Verabreichung und/oder eine längerfristige oder Dauerbehandlung notwendig ist. Insbesondere umfasst der Begriff einer "Akutbehandlung" im Sinne der vorliegenden Erfindung, dass die erfindungsgemäße Zusammensetzung bei Bemerkbarmachen einer Herpes-Infektion eine schnelle, beispielsweise innerhalb eines Tages, Wirkung zeigen oder einen Ausbruch einschließlich einer Bläschenbildung verhindern kann.

Bevorzugte ist eine Darreichungsform ausgewählt aus der Gruppe umfassend Kautabletten, Kaudragees, Lutschtabletten, Pastillen, Schmelztabletten, Gel, Dosierspray und/oder Kaugummi. Es wird angenommen, dass die positive Wirkung der Zusammensetzung insbesondere auf der Darreichungsform ausgewählt aus der Gruppe umfassend Kautabletten, Kaudragees, Lutschtabletten, Pastillen, Schmelztabletten, Gel, Dosierspray und/oder Kaugummi beruht. So kann durch diese Darreichungsformen insbesondere durch eine Kautablette eine sofortige verbesserte Resorption durch Mundschleimhaut zur Verfügung gestellt werden. So löst sich eine Kautablette nach einer Einnahme schnell im Mund auf. Außerdem tritt eine Wirkung so direkt im Mundbereich ein, dort wo auch Lippenherpes auftritt. Insbesondere bewirkt ein Zerkauen eine großräumige Verteilung der Wirkstoffe im Mundbereich. Dies stellt weiterhin einen Vorteil gegenüber einer Verabreichung über Tabletten, Kapseln oder Trinkampullen dar, die unzerkaut heruntergeschluckt werden. Die erfindungsgemäßen Darreichungsformen werden gekaut, gelutscht oder im Mund zergehen gelassen, wobei bereits im Mundraum die Wirkstoffe freigesetzt werden. Durch den Kauvorgang werden diese dadurch in dosierter Form und über den Kauvorgang zunächst weitgehend gleichmäßig resorbierbar zur Verfügung gestellt.

Darüber hinaus ist eine Verabreichung von Tabletten auf Wasser oder andere Getränke angewiesen. Dies ist umständlich, während Darreichungsformen wie Kautabletten, Kaudragees, Lutschtabletten, Pastillen, Schmelztabletten, Gel, Dosierspray oder Kaugummis einfach und praktisch transportiert und angewendet werden können.

Eine Verabreichung von Wirkstoffen über eine Kautablette, Kaudragee, Lutschtablette, Pastille, Schmelztablette, Gel, Dosierspray oder ein Kaugummi kann die Wirkstoffe ferner über eine gewisse Applikationsdauer dem Körper zuführen. Hierbei kann eine Resorption sowohl über die Mundschleimhaut wie auch über den Verdauungstrakt stattfinden, wenn die Wirkstoffe mit dem Speichel heruntergeschluckt werden und in den Magen gelangen. Somit kann sowohl eine wirksame Zuführung der Wirkstoffe über die Mund- und Rachenschleimhaut wie auch durch den Verdauungstrakt erreicht werden. Hierdurch ist über die systemische Aufnahme neben einer Wirkung auf Lippen-Herpes oder Entzündungen des Mund- und Rachenraums auch eine Wirkung auf andere Herpes-Infektionen möglich.

Die erfindungsgemäßen Darreichungsformen sind kaubar, bzw. im Fall der Lutschtabletten, Pastillen und Schmelztabletten lutschbar. Ein Gel kann länger als eine Flüssigkeit im Mund verweilen und sich dort ausbreiten. Gele sind halbfeste Zubereitungen zur Anwendung in der Mundhöhle. Dosiersprays sind dem ähnlich. Kautabletten sind nicht im Arzneibuch als eigenständige Darreichungsform aufgeführt, unterscheiden sich von Lutschtabletten jedoch durch ihr üblicherweise höheres Gewicht, das beispielsweise im Bereich von 1,5 g bis 3 g liegen kann, und eine längere Zerfallszeit. Kaudragees sind mit einem Film überzogen. Schmelztabletten sind nicht überzogene Tabletten, die im Mund behalten werden, wo sie sich schnell verteilen, bevor sie geschluckt werden. Lutschtabletten und Pastillen sind feste, einzeldosierte Zubereitungen, die zum Lutschen bestimmt sind, um üblicherweise eine lokale Wirkung in der Mundhöhle und im Rachen zu erzielen. Sie lösen sich beim Lutschen langsam auf oder zerfallen. Wirkstoffhaltige Kaugummis sind feste Einzeldosiszubereitungen mit einer Grundmasse, die vorwiegend aus Gummi besteht. Sie sind zum Kauen, jedoch nicht zum Schlucken bestimmt. Die Wirkstoffe werden beim Kauen freigesetzt. Übliche und physiologisch akzeptable Grund- und Hilfsstoffe zur Herstellung der Darreichungsformen sind dem Fachmann bekannt.

Unter dem Begriff "Tagesdosis" wird im Sinne dieser Erfindung die Menge der Zusammensetzung verstanden, die pro Tag zugeführt wird, wobei die Stoffmenge, die pro Tag zugeführt werden sollte innerhalb der jeweils angegebenen Mengenbereiche der jeweiligen Stoffe liegen kann. Der Begriff der Tagesdosis kann als Tagesverzehrsmenge verstanden werden.

Unter dem Begriff "Stoff' sind im Sinne dieser Erfindung die Stoffe zu verstehen, die die Zusammensetzung aufweist, insbesondere ausgewählt aus der Gruppe umfassend Zink, Lysin, Selen, Vitamin B₂, Vitamin B₆, Vitamin B₁₂, Vitamin C, Vitamin D₃, Folsäure und/oder Bioflavonoide.

Angaben in mg bzw. µg bezogen auf Zink wie auch auf Selen sind jeweils auf das Metall bezogen, wobei diese beispielsweise in Form anorganischer und/oder organischer Salze enthalten sein können. Zink ist vorzugsweise in Form anorganischer und/oder organischer Salze enthalten. Bevorzugte anorganische Salze sind ausgewählt aus der Gruppe umfassend Zinkchlorid, Zinkoxid, Zinksulfat und/oder Zinkhydroxid. Bevorzugte organische Salze sind ausgewählt aus der Gruppe umfassend Zink-Acetat, Zink-Aspartat, Zink-Gluconat, Zink-Orotat und/oder Zink-Lactat.

Der menschliche Körper verarbeitet Aminosäuren in ihrer L-Form. Daher wird der Begriff "Lysin" im Sinne dieser Erfindung synonym zu "L-Lysin" verwendet. L-Lysin ist eine körpereigene Aminosäure.

Von Vorteil ist, dass die erfindungsgemäß enthaltenen Mengen an Lysin und Zink kann nicht zu einer Überdosierung führen. In bevorzugten Ausführungsformen umfasst die Zusammensetzung, bezogen auf eine Tagesdosis der Zusammensetzung, als wirksame Bestandteile:
- L-Lysin im Bereich von ≥ 1,5 g bis ≤ 6 g, vorzugsweise 3 g, und
- Zink im Bereich von ≥ 15 mg bis ≤ 60 mg, vorzugsweise 30 mg.

Diese Tagesdosierungen können einen Herpes-Ausbruch besonders gut unterdrücken bzw. eine Verkürzung der Infektionsdauer zur Verfügung stellen. Auch im Rahmen einer Prophylaxe von Herpes-Infektionen und/oder Entzündungen des Mund- und Rachenraums können diese Tagesdosierungen einen Herpes-Ausbruch gut unterdrücken.

Die Zusammensetzung ist geeignet zur nutritiven Ergänzung oder prophylaktischen und/oder therapeutischen diätetischen Behandlung bei Herpes-Infektionen und/oder Entzündungen des Mund- und Rachenraums.

Unter dem Begriff "Herpes-Infektionen" werden im Sinne dieser Erfindung Infektionen verstanden, die durch humanpathogene Herpes-Viren wie die Herpes simplex-Viren, das Varicella Zoster-Virus, das Epstein-Barr-Virus, das Cytomegalie-Virus, sowie das humane Herpes-Virus Typ 6 verursacht werden. Herpes-Infektion sind vorzugsweise ausgewählt aus der Gruppe umfassend Lippenherpes (Herpes labialis), Mundfäule (Gingivostomatitis herpetica), Herpes der Geschlechtsorgane (Herpes genitalis), Windpocken oder Gürtelrose (Herpes zoster), Pfeiffersches Drüsenfieber, Speicheldrüsenkrankheit und/oder Drei-Tage-Fieber. Bevorzugte Herpes-Infektion sind durch Herpes simplex-Viren insbesondere Herpes-Virus Typ 1 verursachte Infektionen, insbesondere Lippenherpes (Herpes labialis) und Mundfäule (Gingivostomatitis herpetica).

Die Mundfäule oder Gingivostomatitis herpetica, auch aphthöse Stomatitis, Stomatitis aphthosa oder Stomatitis herpetica genannt, ist eine durch das Herpes-Virus Herpes simplex Typ 1 (HSV-1) ausgelöste Erkrankung der Mundschleimhaut und des Zahnfleischs.

Unter dem Begriff "Entzündungen des Mund- und Rachenraums" werden im Sinne dieser Erfindung Entzündungen verstanden, die den Mund- und Rachenraum betreffen, einschließlich des Zahnfleischs und der Rachenschleimhaut. Entzündungen des Mund- und Rachenraums sind insbesondere virale Entzündungen, können jedoch auch durch Bakterien verursachte Entzündungen umfassen.

Bevorzugt sind Entzündungen des Mund- und Rachenraums ausgewählt aus der Gruppe umfassend Gingivitis, Stomatitis und/oder Pharyngitis. Unter dem Begriff "Gingivitis" werden Entzündungen des marginalen Zahnfleischs (Gingiva) verstanden. Zahnfleischentzündungen werden zumeist durch Bakterien ausgelöst, können jedoch auch durch Viren ausgelöst werden. Unter dem Begriff "Stomatitis" werden Entzündungen der Mundschleimhaut verstanden. Entzündungen der Mundschleimhaut können infektiös durch Viren ausgelöst werden. Unter dem Begriff "Pharyngitis" oder "Rachenentzündung" werden Entzündungen der Rachenschleimhaut verstanden. Eine Pharyngitis kann von Influenza- oder Adenoviren verursacht werden, jedoch auch durch Herpes simplex-Viren. Auch das Epstein-Barr-Virus sowie das Cytomegalie-Virus können eine Rachentzündung auslösen.

Die Zusammensetzung kann weitere wirksame Bestandteile enthalten. In einigen Ausführungsformen kann die Zusammensetzung wirksame Bestandteile ausgewählt aus der Gruppe umfassend Selen, Vitamin B₂, Vitamin B₆, Vitamin B₁₂, Vitamin C, Vitamin D₃, Folsäure und/oder Bioflavonoide umfassen. Vorteilhaft im Sinn der Erfindung kann insbesondere eine Kombination mit Selen, Vitamin B₂, Vitamin B₆, Vitamin B₁₂, Vitamin C, Vitamin D₃, Folsäure und Bioflavonoiden sein. Diese können einen weiter verbesserten Schutz vor Herpes-Infektionen und/oder Entzündungen des Mund- und Rachenraums zur Verfügung stellen. Eine Kombination mit Selen, Vitamin B₂, Vitamin B₆, Vitamin B₁₂, Vitamin C, Vitamin D₃, Folsäure und/oder Bioflavonoiden kann insbesondere zu einer allgemeinen Unterstützung eines intakten antiviralen Abwehrmechanismus beitragen.

Unter dem Begriff "Bioflavonoide" werden im Sinne dieser Erfindung natürliche Flavonoide erhältlich Pflanzen verstanden. Diese können beispielsweise aus der Schalenhaut von Zitrusfrüchten gewonnen werden. Unter dem Begriff "Citrusbioflavonoide" werden im Sinne dieser Erfindung natürliche Flavonoide erhalten aus der Schalenhaut von Zitrusfrüchten verstanden. Zu den Bioflavonoiden zählen beispielsweise Hesperidin, Rutin und Quercitin. Selen ist vorzugsweise in Form von Selenit, Selenat und/oder Selenhefe enthalten. Vitamin C kann in natürlicher und/oder synthetischer Form enthalten sein. Unter "natürlichem Vitamin C" wird Vitamin C in "nahrungsgebundener" Form verstanden, beispielsweise als Citruskonzentrat. Vitamin C kann ferner in Form von isolierter Ascorbinsäure oder isoliertem Mineralascorbat verstanden, das meist synthetisch hergestellt wird.

In bevorzugten Ausführungsformen kann die Zusammensetzung bezogen auf eine Tagesdosis der Zusammensetzung als wirksame Bestandteile umfassen:
- Selen im Bereich von ≥ 10 µg bis ≤ 300 µg, vorzugsweise im Bereich von ≥ 50 µg bis ≤ 200 µg, bevorzugt 100 µg; und/oder
- Vitamin B₂ im Bereich von ≥ 1,8 mg bis ≤ 100 mg, vorzugsweise im Bereich von ≥ 4,5 mg bis ≤ 50 mg, bevorzugt 6 mg; und/oder
- Vitamin B₆ im Bereich von ≥ 1,8 mg bis ≤ 100 mg, vorzugsweise im Bereich von ≥ 4,5 mg bis ≤ 50 mg, bevorzugt 6 mg; und/oder
- Vitamin B₁₂ im Bereich von ≥ 1 µg bis ≤ 100 µg, vorzugsweise im Bereich von ≥ 2 µg bis ≤ 30 µg, bevorzugt 6 µg; und/oder
- Vitamin C im Bereich von ≥ 25 mg bis ≤ 2 g, vorzugsweise im Bereich von ≥ 50 mg bis ≤ 1 g, bevorzugt 500 mg; und/oder
- Vitamin D₃ im Bereich von ≥ 1 µg bis ≤ 50 µg, vorzugsweise im Bereich von ≥ 2,5 µg bis ≤ 20 µg, bevorzugt 5 µg; und/oder
- Folsäure im Bereich von ≥ 50 µg bis ≤ 2 mg, vorzugsweise im Bereich von ≥ 100 µg bis ≤ 1 mg, bevorzugt 500 µg; und/oder
- Bioflavonoide im Bereich von ≥ 1 mg bis ≤ 100 mg, vorzugsweise im Bereich von ≥ 2,5 mg bis ≤ 50 mg, bevorzugt 5 mg.

Vorteilhaft ist insbesondere, dass die Dosierungen der erfindungsgemäßen Zusammensetzung keine Nebenwirkungen verursachen. Dies ermöglicht, die Zusammensetzung nicht nur bei beginnendem Juckreiz, Schmerzgefühl oder Bläschenbildung an den Lippen zu verwenden, sondern bereits bei Anzeichen Herpes-auslösender Faktoren wie Erkältung, Sonneneinstrahlung, Stress oder Ekelgefühlen.

In Ausführungsformen enthält die erfindungsgemäße Zusammensetzung pro 100 g der Zusammensetzung 21 g L-Lysin, 213 mg Zink, 709 mg Selen, 43 mg Vitamin B2, 43 mg Vitamin B6, 43 mg Vitamin B12, 3547 mg Vitamin C, 35 mg Vitamin D3, 3547 mg Folsäure und 36 mg Citrusbioflavonoide.

Die Zusammensetzung liegt vorzugsweise fest- oder gelförmig vor. Unter den Darreichungsform ausgewählt aus der Gruppe umfassend Kautablette, Kaudragee, Lutschtablette, Pastille, Schmelztablette, Gel, Dosierspray und/oder Kaugummi, sind Kautabletten, Kaudragees und Lutschtabletten, insbesondere Kautabletten, bevorzugt. Insbesondere durch eine Kautablette kann eine sofortige verbesserte Resorption durch die Mundschleimhaut zur Verfügung gestellt werden. So löst sich eine Kautablette nach einer Einnahme schnell im Mund auf. Außerdem tritt eine Wirkung so direkt im Mundbereich ein, dort wo auch Lippenherpes auftritt. Insbesondere bewirkt das Zerkauen einer Kautablette eine großräumige Verteilung der Wirkstoffe im Mundbereich. Zudem werden diese durch den Kauvorgang in dosierter Form und weitgehend gleichmäßig resorbierbar zur Verfügung gestellt. Kautabletten haben den weiteren Vorteil, dass sie sehr einfach ohne Wasser zerkaut werden können. In bevorzugten Ausführungsformen liegt die Zusammensetzung daher in Form einer Kautablette vor.

Eine Darreichungsform kann die Stoffe und/oder die Stoffgewichtsgehalte in der Menge einer Tagesdosis enthalten. Weiterhin kann vorgesehen sein, dass die Tagesdosis auf eine oder mehrere Einzeldosen verteilt verabreichbar ist, beispielsweise auf zwei, drei, vier, fünf oder sechs Einzeldosen. Die Tagesdosis und/oder Einzeldosis kann auch auf mehrere gleiche oder unterschiedliche Präparatformen verteilt sein, wobei die Präparatformen gleiche oder unterschiedliche Stoffe und/oder Stoffgewichtsgehalte aufweisen können.

Vorteilhaft bei einer Verabreichung der enthaltenen Stoffe in mehreren getrennten Dosen ist insbesondere eine Verlängerung Wirkdauer insgesamt im Mundbereich. Die Tagesdosis der Zusammensetzung kann in Form von 1 bis 6 Einzeldosen, vorzugsweise 2 bis 5 Einzeldosen, insbesondere 3 oder 4 Einzeldosen vorliegen. Die Tagesdosis der Zusammensetzung kann insbesondere in Form von 1 bis 6, vorzugsweise 2 bis 5, insbesondere 3 oder 4 Kautabletten, Kaudragees, Lutschtabletten, Pastillen, Schmelztabletten, Gel- oder Dosiersprayportionen und/oder Kaugummis vorliegen. Bevorzugt liegt eine Tagesdosis in Form von 3 Kautabletten vor. Weiter bevorzugt liegt eine Tagesdosis in Form von vier Kautabletten vor.

Unter einer "Einzeldosis" wird im Sinne dieser Anmeldung eine Verabreichungseinheit der Zusammensetzung verstanden. Eine Einzeldosis entspricht beispielsweise einer Kautablette, einem Kaudragee, einer Lutschtablette, einer Pastille, einer Schmelztablette, einer Portion Gel oder Spray oder einem Kaugummi.

In bevorzugten Ausführungsformen umfasst die Zusammensetzung bezogen auf eine Einzeldosis der Zusammensetzung als wirksame Bestandteile:
- L-Lysin im Bereich von ≥ 250 mg bis ≤ 3 g, vorzugsweise im Bereich von ≥ 500 mg bis ≤ 2 g, bevorzugt 1 g, und
- Zink im Bereich von ≥ 3 mg bis ≤ 30 mg, vorzugsweise im Bereich von ≥ 5 mg bis ≤ 20 mg, bevorzugt 10 mg.

In weiter bevorzugten Ausführungsformen kann die Zusammensetzung bezogen auf eine Einzeldosis der Zusammensetzung als wirksame Bestandteile umfassen:
- Selen im Bereich von ≥ 3,3 µg bis ≤ 100 µg, vorzugsweise im Bereich von ≥ 16,7 µg bis ≤ 66,7 µg, bevorzugt 33,3 µg; und/oder
- Vitamin B₂ im Bereich von ≥ 0,6 mg bis ≤ 33,3 mg, vorzugsweise im Bereich von ≥ 1,5 mg bis ≤ 16,7 mg, bevorzugt 2 mg; und/oder
- Vitamin B₆ im Bereich von ≥ 0,6 mg bis ≤ 33,3 mg, vorzugsweise im Bereich von ≥ 1,5 mg bis ≤ 16,7 mg, bevorzugt 2 mg; und/oder
- Vitamin B₁₂ im Bereich von ≥ 0,3 µg bis ≤ 33,3 µg, vorzugsweise im Bereich von ≥ 0,7 µg bis ≤ 10 µg, bevorzugt 2 µg; und/oder
- Vitamin C im Bereich von ≥ 8,3 mg bis ≤ 666,7 mg, vorzugsweise im Bereich von ≥ 16,7 mg bis ≤ 333,3 mg, bevorzugt 166,7 mg; und/oder
- Vitamin D₃ im Bereich von ≥ 0,3 µg bis ≤ 16,7 µg, vorzugsweise im Bereich von ≥ 0,8 µg bis ≤ 6,7 µg, bevorzugt 1,7 µg; und/oder
- Folsäure im Bereich von ≥ 16,7 µg bis ≤ 666,7 µg, vorzugsweise im Bereich von ≥ 33,3 µg bis ≤ 333,3 µg, bevorzugt 166,7 µg; und/oder
- Bioflavonoide im Bereich von ≥ 0,3 mg bis ≤ 33,3 mg, vorzugsweise im Bereich von ≥ 0,8 mg bis ≤ 16,7 mg, bevorzugt 1,7 mg.

Die Zusammensetzung kann ein funktionelles Lebensmittel, diätetisches Lebensmittel, ergänzende bilanzierte Diät und/oder ein Nahrungsergänzungsmittel sein. Insbesondere ist bevorzugt, dass die Zusammensetzung ein diätetisches Lebensmittel für besondere medizinische Zwecke, insbesondere für eine bilanzierte Diät, ist.

Ein weiterer Gegenstand betrifft daher Lebensmittel, insbesondere funktionelle Lebensmittel, diätetische Lebensmittel, Mittel zur ergänzenden bilanzierten Diät und/oder Nahrungsergänzungsmittel, umfassend als wirksame Bestandteile die Stoffe der Zusammensetzung, insbesondere, bezogen auf eine Tagesdosis des Lebensmittels, L-Lysin im Bereich von ≥ 750 mg bis ≤ 9 g, und Zink im Bereich von ≥ 9 mg bis ≤ 90 mg, wobei das Lebensmittel in einer Darreichungsform ausgewählt aus der Gruppe umfassend Kautabletten, Kaudragees, Lutschtabletten, Pastille, Schmelztablette, Gel, Dosierspray und/oder Kaugummi vorliegt. Gegebenenfalls kann das Lebensmittel weiterhin Selen, Vitamin B₂, Vitamin B₆, Vitamin B₁₂, Vitamin C, Vitamin D₃, Folsäure und/oder Bioflavonoide umfassen.

Durch die vorteilhaften Stoffe der Zusammensetzung ist diese insbesondere im Rahmen einer diätetischen Ernährungsberatung als Nahrungsergänzungsmittel oder Mittel zur ergänzenden bilanzierten Diät verwendbar. Die Zusammensetzung ist insbesondere geeignet zur Verwendung als Mittel zur ergänzenden bilanzierten Diät und/oder als Mittel zur Nahrungsergänzung zur Prophylaxe und/oder Behandlung von Herpes-Infektionen und/oder Entzündungen des Mund- und Rachenraums.

Bevorzugt ist eine Verwendung der erfindungsgemäßen Zusammensetzung zur Herstellung eines funktionellen Lebensmittels, diätetischen Lebensmittels, ergänzenden bilanzierten Diät und/oder Nahrungsergänzungsmittels, insbesondere zur nutritiven Ergänzung oder prophylaktischen und/oder therapeutischen Behandlung von Herpes-Infektionen insbesondere von Lippenherpes (Herpes labialis) oder Mundfäule (Gingivostomatitis herpetica) und/oder Entzündungen des Mund- und Rachenraums, insbesondere ausgewählt aus der Gruppe umfassend Gingivitis, Stomatitis und/oder Pharyngitis.
Besonders bevorzugt ist eine Verwendung in der Akutbehandlung von Herpes-Infektionen und/oder Entzündungen des Mund- und Rachenraums, insbesondere in der Akutbehandlung von Lippenherpes.

Herpes-Infektion sind vorzugsweise ausgewählt aus der Gruppe umfassend Lippenherpes (Herpes labialis), Mundfäule (Gingivostomatitis herpetica), Herpes der Geschlechtsorgane (Herpes genitalis), Windpocken oder Gürtelrose (Herpes zoster), Pfeiffersches Drüsenfieber, Speicheldrüsenkrankheit und/oder Drei-Tage-Fieber. Bevorzugte Herpes-Infektion sind durch Herpes simplex-Viren insbesondere Herpes-Virus Typ 1 verursachte Infektionen, insbesondere Lippenherpes (Herpes labialis) und Mundfäule (Gingivostomatitis herpetica).

Bevorzugte Entzündungen des Mund- und Rachenraums sind ausgewählt aus der Gruppe umfassend Gingivitis, Stomatitis und/oder Pharyngitis.

Insbesondere ist die erfindungsgemäße Zusammensetzung geeignet zur nutritiven Ergänzung oder prophylaktischen und/oder therapeutischen diätetischen Behandlung bei Herpes-Infektionen und/oder Entzündungen des Mund- und Rachenraums.

Beispiele für Zusammensetzungen, die der Veranschaulichung der vorliegenden Erfindung dienen, sind nachstehend angegeben.

Es versteht sich von selbst, dass die Darreichungsformen, ausgewählt aus der Gruppe umfassend Kautabletten, Kaudragees, Lutschtabletten, Pastille, Schmelztablette, Gel, Dosierspray und/oder Kaugummi, die üblichen zur Formulierung der jeweiligen Darreichungsforen eingesetzten Hilfsstoffe aufweisen, so dass in den Beispielen lediglich die enthaltenen Wirkstoffe aufgeführt sind.

### Beispiel 1

Eine Kautablette enthaltend 1 g L-Lysin und 10 mg Zink.

### Beispiel 2

Eine Kautablette enthaltend die folgenden Stoffe:
- 1 g L-Lysin
- 10 mg Zink
- 33,3 µg Selen
- 2 mg Vitamin B₂
- 2 mg Vitamin B₆
- 2 µg Vitamin B₁₂
- 166,7 mg Vitamin C
- 1,7 µg Vitamin D₃
- 166,7 µg Folsäure
- 1,7 mg Bioflavonoide.

### Beispiel 3

Zusammensetzung umfassend 3 Kautabletten enthaltend insgesamt folgende Stoffe:
- 3 g L-Lysin
- 30 mg Zink.

### Beispiel 4

Zusammensetzung umfassend 3 Kautabletten enthaltend insgesamt folgende Stoffe:
- 3 g L-Lysin
- 30 mg Zink
- 100 µg Selen
- 6 mg Vitamin B₂
- 6 mg Vitamin B₆
- 6 µg Vitamin B₁₂
- 500 mg Vitamin C
- 5 µg Vitamin D₃
- 500 µg Folsäure
- 5 mg Bioflavonoide.

### Beispiel 5

Ein Kaudragee enthaltend 1 g L-Lysin und 10 mg Zink.

### Beispiel 6

Eine Lutschtablette enthaltend 1 g L-Lysin und 10 mg Zink.

### Beispiel 7

Ein Kaugummi enthaltend 1 g L-Lysin und 10 mg Zink.

### Beispiel 8

Ein Beutel enthaltend eine Portion Gel enthaltend 1 g L-Lysin und 10 mg Zink.

### Beispiel 9

Die Wirkung der erfindungsgemäßen im Beispiel 4 beschriebenen Zusammensetzung umfassend 3 Kautabletten wurde bei Patienten mit wiederkehrenden Lippenherpes-Infektionen beobachtet. Hierbei wurde bei Anzeichen von Juckreiz, Kribbeln oder Schmerzgefühl an den Lippen für zwei bis fünf Tage drei Mal täglich eine Kautablette verabreicht.

Es konnte festgestellt werden, dass durch die Verabreichung der erfindungsgemäßen Zusammensetzung gemäß Beispiel 4 bereits nach zwei bis drei Tagen eine deutliche Verringerung der Häufigkeit mit der daraufhin ein Lippenherpes ausbrach erzielt werden konnte. Auch bei wiederholter und/oder langfristiger Einnahme waren keine Gegenanzeigen oder Nebenwirkungen zu beobachten, insbesondere traten keine Unverträglichkeiten auf.

### Beispiel 10

Die Wirkung der erfindungsgemäßen Zusammensetzung wurde in einer Anwendungsstudie umfassend 64 Teilnehmer mit wiederkehrenden Lippenherpes-Infektionen beobachtet, wobei die Studienteilnehmer wenigstens ein bis sechsmal oder häufiger Lippenherpes pro Jahr hatten. Hierbei nahmen 31 Teilnehmer ausschließlich die Kautablette, während 33 Teilnehmer die Kautablette als zusätzliche Therapie neben weiteren Mitteln wie Aciclovir- oder Zink-Creme, Lippenpatches oder Immunstimulantien, zur Behandlung des Herpes einnahmen.

Die Studienteilnehmer erhielten Kautabletten enthaltend 1 g L-Lysin, 10 mg Zink, 33,3 µg Selen, 2 mg Vitamin B₂, 2 mg Vitamin B₆, 2 µg Vitamin B₁₂, 166,7 mg Vitamin C, 1,7 µg Vitamin D₃, 166,7 µg Folsäure und 1,7 mg Bioflavonoide. Bei Anzeichen von Juckreiz, Kribbeln oder Schmerzgefühl an den Lippen nahmen die Teilnehmer täglich jeweils drei Kautabletten, wobei die Einnahme über fünf Tage erfolgte.

Bei den Teilnehmern, die ausschließlich die Kautablette einnahmen, stellten eine merkliche Verbesserung der Symptome nach durchschnittlich 2,5 Tagen fest, während bisherige, herkömmliche Therapien eine Besserung erst nach 4,8 Tagen zeigten. Teilnehmer, die die Kautablette als zusätzliche Therapie einnahmen, zeigten durchschnittlich eine merkliche Verbesserung der Symptome nach 4,2 Tagen gegenüber 4,8 Tagen fest.

Ein Teil der Teilnehmer, die bei Einsetzen von Juckreiz oder Schmerzgefühl an den Lippen ausschließlich die Kautablette einnahmen, stellten keinen Ausbruch der Herpes-Infektion durch Bläschenbildung fest. Ein weiterer Teil der Teilnehmer, die nur die Kautablette einnahmen, stellten fest, dass sie am nächsten Tag bereits fast symptomfrei waren. Dies zeigt, dass bei einer rechtzeitigen Einnahme der Zusammensetzung ein Ausbruch der Herpes-Infektion unterdrückt werden konnte. Insbesondere zeigt dies, dass eine Verabreichung der erfindungsgemäßen Zusammensetzung im Sinne einer Akutbehandlung eine Wirkung erzielen kann. Weiterhin war der Herpes bei 17 der Teilnehmer, die ausschließlich die Kautablette einnahmen, und 11 der Teilnehmer, die die Kautablette als zusätzliche Therapie zur Behandlung des Herpes einnahmen, spätestens am fünften Tag vollständig abgeheilt. Demgegenüber waren mit herkömmlichen Therapien lediglich vier Teilnehmer am fünften Tag symptomfrei.

Insbesondere wurde nicht nur die Wirksamkeit, sondern auch der Geruch der Kautabletten als sehr gut empfunden.

Dies zeigt, dass eine Verwendung der erfindungsgemäßen Zusammensetzung die Symptome einer Herpes-Infektion schnell reduzieren kann und insbesondere für eine Akuttherapie geeignet ist. Weiterhin konnte festgestellt werden, eine Verbesserung auch als Zusatztherapie zu anderen Herpes-Therapeutika festgestellt werden konnte.

## Patentansprüche

1. Zusammensetzung zur Verwendung in der prophylaktischen und/oder therapeutischen Behandlung von Lippenherpes, **dadurch gekennzeichnet, dass** die Zusammensetzung, bezogen auf eine Verabreichungseinheit der Zusammensetzung, als wirksame Bestandteile die nachstehenden Stoffe aufweist:
- L-Lysin im Bereich von ≥ 250 mg bis ≤ 3 g, und
- Zink im Bereich von ≥ 3 mg bis ≤ 30 mg, und
- Selen, Vitamin B₂, Vitamin B₆, Vitamin B₁₂, Vitamin C, Vitamin D₃, Folsäure und Bioflavonoide,
und in einer Darreichungsform ausgewählt aus der Gruppe umfassend Kautabletten, Kaudragees, Lutschtabletten, Pastille, Schmelztablette, Gel und/oder Kaugummi vorliegt, wobei die Darreichungsform gekaut, gelutscht oder im Mund zergehen gelassen wird, wobei die Wirkstoffe im Mundraum freigesetzt werden.

2. Zusammensetzung zur Verwendung nach Anspruch 1, **dadurch gekennzeichnet, dass** die Zusammensetzung in der Akutbehandlung von Lippen-Herpes verwendet wird.

3. Zusammensetzung zur Verwendung nach Anspruch 1 oder 2, **dadurch gekennzeichnet, dass** von 1 bis 6, vorzugsweise 2 bis 5, insbesondere 3 oder 4, Verabreichungseinheiten der Zusammensetzung pro Tag zugeführt werden.

4. Zusammensetzung zur Verwendung nach einem der vorherigen Ansprüche, **dadurch gekennzeichnet, dass** die Zusammensetzung in Form einer Kautablette vorliegt.

5. Zusammensetzung zur Verwendung nach einem der vorherigen Ansprüche, **dadurch gekennzeichnet, dass** die Zusammensetzung bezogen auf eine Verabreichungseinheit der Zusammensetzung als wirksame Bestandteile umfasst:
- L-Lysin im Bereich von ≥ 500 mg bis ≤ 2 g, bevorzugt 1 g, und
- Zink im Bereich von ≥ 5 mg bis ≤ 20 mg, bevorzugt 10 mg.

6. Zusammensetzung zur Verwendung nach einem der vorherigen Ansprüche, **dadurch gekennzeichnet, dass** die Zusammensetzung bezogen auf eine Verabreichungseinheit der Zusammensetzung als wirksame Bestandteile umfasst:
- Selen im Bereich von ≥ 3,3 µg bis ≤ 100 µg; und/oder
- Vitamin B₂ im Bereich von ≥ 0,6 mg bis ≤ 33,3 mg; und/oder
- Vitamin B₆ im Bereich von ≥ 0,6 mg bis ≤ 33,3 mg; und/oder
- Vitamin B₁₂ im Bereich von ≥ 0,3 µg bis ≤ 33,3 µg; und/oder
- Vitamin C im Bereich von ≥ 8,3 mg bis ≤ 666,7 mg; und/oder
- Vitamin D₃ im Bereich von ≥ 0,3 µg bis ≤ 16,7 µg; und/oder
- Folsäure im Bereich von ≥ 16,7 µg bis ≤ 666,7 µg; und/oder
- Bioflavonoide im Bereich von ≥ 0,3 mg bis ≤ 33,3 mg.

7. Zusammensetzung zur Verwendung nach einem der vorherigen Ansprüche, **dadurch gekennzeichnet, dass** die Zusammensetzung ein funktionelles Lebensmittel, diätetisches Lebensmittel, ergänzende bilanzierte Diät und/oder Nahrungsergänzungsmittel ist.

## Claims

1. Composition for use in the prophylactic and/or therapeutic treatment of cold sores, **characterized in that** the composition comprises, based on a dosage unit of the composition, as active components the substances below:
- L-lysine in the range from ≥ 250 mg to ≤ 3 g, and
- zinc in the range from ≥ 3 mg to ≤ 30 mg, and
- selenium, vitamin B₂, vitamin B₆, vitamin B₁₂, vitamin C, vitamin D₃, folic acid and bioflavonoids,
and is present in an administration form selected from the group comprising chewable tablets, chewable coated pills, lozenges, pastilles, orally disintegrating tablets, gels and/or chewing gums, wherein the administration forms are chewed, sucked or allowed to disintegrate in the mouth, with the active compounds being released in the oral cavity.

2. The composition for use according to claim 1, **characterized in that** the composition is used in the acute treatment of cold sores.

3. The composition for use according to claim 1 or 2, **characterized in that** from 1 to 6, preferably 2 to 5, particularly 3 to 4, dosage units of the composition are administered per day.

4. The composition for use according to any of the preceding claims, **characterized in that** the compositionis is present in form of a chewable tablet.

5. The composition for use according to any of the preceding claims, **characterized in that** the composition comprises, based on a dosage unit of the composition, as active components:
- L-lysine in the range from ≥ 500 mg to ≤ 2 g, preferably 1 g, and
- zinc in the range from ≥ 5 mg to ≤ 20 mg, preferably 10 mg.

6. The composition for use according to any of the preceding claims, **characterized in that** the composition comprises, based on a dosage unit of the composition, as active components:
- selenium in the range from ≥ 3.3 µg to ≤ 100 µg; and/or
- vitamin B₂ in the range from ≥ 0.6 mg to ≤ 33.3 mg; and/or
- vitamin B₆ in the range from ≥ 0.6 mg to ≤ 33.3 mg; and/or
- vitamin B₁₂ in the range from ≥ 0.3 µg to ≤ 33.3 µg; and/or
- vitamin C in the range from ≥ 8.3 mg to ≤ 666.7 mg; and/or
- vitamin D₃ in the range from ≥ 0.3 µg to ≤ 16.7 µg; and/or
- folic acid in the range from ≥ 16.7 µg to ≤ 666.7 µg; and/or
- bioflanovoids in the range from ≥ 0.3 mg to ≤ 33.3 mg.

7. The composition for use according to any of the preceding claims, **characterized in that** the composition is a functional food, dietary food, supplementary balanced diet and/or food supplement.

## Revendications

1. Composition pour une utilisation dans le traitement prophylactique et/ou thérapeutique de l'herpès labial, **caractérisée en ce que** la composition présente, par rapport à une dose unitaire de la composition, en tant que constituants actifs, les substances suivantes :
- de la L-lysine dans la plage de ≥ 250 mg à ≤ 3 g et
- du zinc dans la plage de ≥ 3 mg à ≤ 30 mg et
- du sélénium, de la vitamine B₂, de la vitamine B₆, de la vitamine B₁₂, de la vitamine C, de la vitamine D₃, de l'acide folique et des bioflavonoïdes,
et se trouve sous une forme d'administration choisie dans le groupe comprenant les comprimés à mâcher, les dragées à mâcher, les pastilles à sucer, les pastilles, les comprimés orodispersibles, un gel et/ou une gomme à mâcher, la forme d'administration étant mâchée, sucée ou laissée à fondre en bouche, les principes actifs étant libérés dans la cavité buccale.

2. Composition pour une utilisation selon la revendication 1, **caractérisée en ce que** la composition est utilisée dans le traitement aigu de l'herpès labial.

3. Composition pour une utilisation selon la revendication 1 ou 2, **caractérisée en ce que** 1 à 6, de préférence 2 à 5, en particulier 3 ou 4 doses unitaires de la composition sont administrées par jour.

4. Composition pour une utilisation selon l'une quelconque des revendications précédentes, **caractérisée en ce que** la composition se trouve sous la forme d'un comprimé à mâcher.

5. Composition pour une utilisation selon l'une quelconque des revendications précédentes, **caractérisée en ce que** la composition comprend, par rapport à une dose unitaire de la composition, en tant que constituants actifs :
- de la L-lysine dans la plage de ≥ 500 mg à ≤ 2 g, de préférence 1 g, et
- du zinc dans la plage de ≥ 5 mg à ≤ 20 mg, de préférence 10 mg.

6. Composition pour une utilisation selon l'une quelconque des revendications précédentes, **caractérisée en ce que** la composition comprend, par rapport à une dose unitaire de la composition, en tant que constituants actifs :
- du sélénium dans la plage de ≥ 3,3 µg à ≤ 100 µg ; et/ou
- de la vitamine B₂ dans la plage de ≥ 0,6 mg à ≤ 33,3 mg ; et/ou
- de la vitamine B₆ dans la plage de ≥ 0,6 mg à ≤ 33,3 mg ; et/ou
- de la vitamine B₁₂ dans la plage de ≥ 0,3 µg à ≤ 33,3 µg ; et/ou
- de la vitamine C dans la plage de ≥ 8,3 mg à ≤ 666,7 mg ; et/ou
- de la vitamine D₃ dans la plage de ≥ 0,3 µg à ≤ 16,7 µg ; et/ou
- de l'acide folique dans la plage de ≥ 16,7 µg à ≤ 666,7 µg ; et/ou
- des bioflavonoïdes dans la plage de ≥ 0,3 mg à ≤ 33,3 mg.

7. Composition pour une utilisation selon l'une quelconque des revendications précédentes, **caractérisée en ce que** la composition est un aliment fonctionnel, un aliment diététique, un régime équilibré complémentaire et/ou un complément alimentaire.
